# EUROPEAN PATENT SPECIFICATION

(11) **EP 0 800 384 B1**
(45) Date of publication and mention of the grant of the patent: **07.03.2001**
(21) Application number: 95942265.0
(22) Date of filing: 14.12.1995
(51) Int. Cl.: A61K 9/20, A61K 31/40, A61K 31/215, A61K 31/19, A61K 31/135

(54) **PHARMACEUTICAL TABLET FORMULATIONS FOR DIRECT COMPRESSION**
PHARMAZEUTISCHE ZUBEREITUNG ZUR DIREKTVERPRESSUNG
FORMULATIONS DE COMPRIMES PHARMACEUTIQUES ELABORES PAR COMPRESSION DIRECTE

(30) Priority: 14.12.1994 IE 940969; 14.12.1994 IE 940971; 31.07.1995 IE 950583; 31.07.1995 IE 950584; 31.07.1995 IE 950586
(43) Date of publication of application: 15.10.1997
(60) Divisional application: 00115840.1
(73) Proprietor: ENBALT TRADING LIMITED, Dublin 2 (IE); BIOGLAN IRELAND (R & D) LIMITED, Dublin 2 (IE)
(72) Inventor: McCARTHY, Paul, Clonmel County Tipperary (IE); DONEGAN, Ann, Kilsheelan County Tipperary (IE); PATTISON, John, Southwater West Sussex RG13 7DE (GB); O'SULLIVAN, Richard, Kildare County Kildare (IE)
(74) Representative: Jump, Timothy John Simon
(86) International application number: IE9500062
(87) International publication number: WO9618386

(56) References cited:
- EP-A- 0 298 666
- EP-A- 0 511 767
- EP-A- 0 519 820
- WO-A-92/08463
- WO-A-93/23025
- WO-A-94/22435
- DE-A- 3 941 703
- US-A- 5 358 941

## Description

The present invention relates to a method of preparing a pharmaceutical tablet comprising: preparing a formulation by dry blending an active pharmaceutical ingredient selected from selegiline, oxybutynin, bumetanide, indapamide and pharmaceutically acceptable salts thereof, a direct compression excipient capable of interacting with the active pharmaceutical ingredient and reducing the amount thereof detectable in the pharmaceutical tablet, and an amount of an inhibitor selected from maize starch, a crospovidone based product and carboxymethyl starch, sufficient to reduce said interaction; and forming said tablet from said formulation by direct compression.

UK-A-1,153,578 describes selegiline hydrochloride which is the compound of the formula

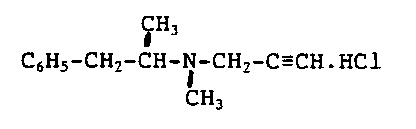

It has 5 monoamine oxidase inhibitor activity and is used in the treatment of Parkinsons disease and as a coronary dilator, hallucinogenic and anti-depressive agent and also as a tranquilliser, analgesic and an appetite suppressant.

It is known to formulate selegiline in tablet form using conventional wet granulation techniques. However, to date it has not been possible to produce a satisfactory tablet composition of selegiline by direct compression techniques. Similar problems have arisen with other active pharmaceutical ingredients.

Direct compression is the preferred technique since it is considered that fewer chemical stability problems are associated with this technique in comparison with the wet granulation process as moisture is considered to be a primary cause of instability in tablet dosage forms. In addition to the advantage of improved active ingredient stability, the use of direct compression makes it unnecessary to use applied heat to dry the damp granule. Other benefits associated with direct compression are related to particle size uniformity.

This invention therefore is directed towards a direct compression pharmaceutical tablet formulation of an active pharmaceutical ingredient.

According to a first aspect of the invention, there is provided a method of preparing a pharmaceutical tablet comprising:
preparing a formulation by dry blending an active pharmaceutical ingredient selected from selegiline, oxybutynin, bumetanide, indapamide and pharmaceutically acceptable salts thereof, a direct compression excipient capable of interacting with the active pharmaceutical ingredient and reducing the amount thereof detectable in the pharmaceutical tablet, and an amount of an inhibitor selected from maize starch, a crospovidone based product and carboxymethyl starch, sufficient to reduce said interaction;
and forming said tablet from said formulation by direct compression.

In a second aspect, the present invention provides a method of preparing a pharmaceutical tablet comprising:
preparing a formulation by dry blending a direct compression excipient comprising direct compression lactose or microcrystalline cellulose, an active pharmaceutical ingredient capable of interacting with direct compression lactose or microcrystalline cellulose such that the amount of said active pharmaceutical ingredient detected in the pharmaceutical tablet is reduced, and an amount of an inhibitor selected from maize starch, a crospovidone based product, and carboxymethyl starch sufficient to reduce said interaction;
and forming said tablet from said formulation by direct compression.

In a preferred embodiment of the invention the formulation contains an amount of the inhibitor to substantially prevent the interaction between the direct compression excipient and the active pharmaceutical ingredient.

In one preferred embodiment of the invention the weight ratio of inhibitor to the active pharmaceutical ingredient is from 1:50 to 4:1, ideally from 8:5 to 12:5. Preferably the inhibitor includes maize starch.

In a preferred embodiment of the first aspect of the invention the excipient is direct compression lactose and/or microcrystalline cellulose.

The weight ratio of direct compression lactose to active pharmaceutical ingredient is preferably from 2:1 to 100:1 most preferably from 7:1 to 50:1, ideally from 8:1 to 11:1.

The weight ratio of microcrystalline cellulose to active pharmaceutical ingredient is preferably from 2:1 to 100:1, most preferably from 3:1 to 17:1, ideally from 4:1 to 16:1.

In one embodiment of the invention, the formulation includes a lubricant, preferably from 0.1% to 5%, most preferably approximately 0.25% by weight of the formulation. The lubricant may be magnesium stearate, zinc stearate, calcium stearate, stearic acid or combinations of these materials.

In one embodiment of the invention, the formulation includes an antioxidant or other suitable stabiliser to enhance the stability of the tablet formulation. Preferably the antioxidant is present in an amount of from 0.5% to 2% by weight of the formulation.

In another embodiment of the invention the formulation may include glidants, disintegrants, fillers, wetting agents, stabilisers, binders, or combinations of these materials.

The active pharmaceutical ingredient may be selegiline or a pharmaceutically acceptable salt thereof, preferably selegiline hydrochloride. The active pharmaceutical ingredient may also be other compounds including oxybutynin or a pharmaceutically acceptable salt thereof, preferably oxybutynin chloride; bumetanide or a pharmaceutically acceptable salt thereof; or indapamide or a pharmaceutically acceptable salt thereof, preferably indapamide hemihydrate.

The invention further provides a method for producing tablets of an active pharmaceutical ingredient comprising the steps of:-
mixing the active pharmaceutical ingredient, a lubricant, a direct compression excipient, and an amount of an inhibitor to substantially prevent interaction between the direct compression lactose and the active pharmaceutical ingredient; and
directly compressing the mixture thus formed to form tablets.

Preferably, when the formulation includes a lubricant, the method includes the step of mixing the ingredients except the lubricant and subsequently adding the lubricant.

In a third aspect, the invention relates to a pharmaceutical tablet, comprising an active pharmaceutical ingredient, prepared by a method in accordance with the first or second aspects of the invention.

The invention will be more clearly understood from the following description thereof given by way of example only.

In each case, the formulation is a direct compression formulation. The ingredients were weighed out and all the ingredients except the lubricant were added to a receptacle and dry blended for about 6 minutes. The lubricant (in these cases magnesium stearate) was then added and the mixture is blended for a further 2 minutes. Tablets were then formed from the blend by conventional direct compression techniques.

In Examples A and 1 to 4, selegiline hydrochloride was used as the active ingredient. However, it will be appreciated that selegiline base or any pharmaceutically acceptable salt thereof may be used as the active ingredient. Similar comments apply to the examples referring to other active ingredients. In examples B and 5 oxybutynin chloride was used. In example 9 indapamide hemihydrate was used.

All of the excipients used in the examples are of pharmaceutical grade.

| | |
|---|---|
| Magnesium Stearate | consists mainly of magnesium stearate with variable proportions of magnesium palmitate and/or magnesium oleate. It is a tablet lubricant. |
| Zinc Stearate | consists mainly of zinc stearate with variable proportions of zinc palmitate and zinc oleate. It is a tablet lubricant. |
| Sodium Lauryl Sulfate | an anionic surfactant used as a detergent and wetting agent in tablet formulations. |
| Croscarmellose Sodium | is a cross-linked polymer of carboxymethylcellulose sodium. It is a tablet disintegrant. |
| Sodium Starch Glycolate | is the sodium salt of a poly-α-glucopyranose in which some of the hydroxyl groups are in the form of carboxymethyl ether. It is a tablet disintegrant. |
| Crospovidone | is a water insoluble synthetic cross-linked homopolymer of N-vinyl-2-pyrollidone. It is a tablet disintegrant. |
| Microcrystalline Cellulose | is a purified, partially depolymerised cellulose and is used as a diluent and has some lubricant and disintegrant properties. |
| Direct Compression Lactose (DC Lactose) | is direct compression grade of lactose which is more fluid and more compressible than crystalline or powdered lactose. |

Various proprietary brands of direct compression lactose are commercially available. In the examples given below, the brand of direct compression lactose used was Tablettose 80 available from Meggle GmbH of Germany. We have also achieved similar results using Pharmatose DC11 which is available from DMV International of The Netherlands.

It will be appreciated that various similar formulations to those described below may be prepared by scaling up or down the components of the mixture so that the new formulation is quantitatively proportional to those given below. For example, formulations containing 10 mgs of selegiline may be prepared in this way. Similarly, an oxybutynin formulation may be prepared in this way with 2.5 mg rather than 5 mg of active. In addition, a bumetanide formulation may also be prepared in this way with 1 mg rather than 5 mg of active ingredient.

### EXAMPLE A

A batch of tablets were prepared by direct compression. Each tablet had the following composition:-

| | |
|---|---|
| 5 mg | selegiline hydrochloride |
| 85 mg | DC Lactose |

The tablets gave an assay of 91.8%.

This example illustrates the low assay that results when tablets of selegiline are manufactured by direct compression techniques using direct compression lactose.

### EXAMPLE 1

A batch of tablets were prepared by direct compression. Each tablet had the following composition:-

| | |
|---|---|
| 5 mg | selegiline hydrochloride |
| 52.75 mg | DC Lactose |
| 24 mg | microcrystalline cellulose |
| 8 mg | maize starch |
| 0.25 mg | magnesium stearate |

The tablets gave an assay of 99.5%.

It will be noted that the use of an inhibitor, in this case maize starch, radically improves the assay result.

### EXAMPLE 2

A batch of tablets were prepared by direct compression. Each tablet had the following composition:-

| | |
|---|---|
| 5 mg | selegiline hydrochloride |
| 48.75 mg | DC Lactose |
| 24 mg | microcrystalline cellulose |
| 12 mg | maize starch |
| 0.25 mg | magnesium stearate |

The tablets gave an assay of 97.94%.

Using a larger amount of maize starch and less DC Lactose did not significantly effect the assay.

### EXAMPLE 3

A batch of tablets were prepared by direct compression. Each tablet had the following composition:-

| | |
|---|---|
| 5 mg | selegiline hydrochloride |
| 48.75 mg | DC Lactose |
| 24 mg | microcrystalline cellulose |
| 11 mg | maize starch |
| 1 mg | citric acid |
| 0.25 mg | magnesium stearate |

The tablets gave an assay of 99.6%.

The addition of an antioxidant, in this case citric acid, is to counteract any oxidation reactions in storage.

### EXAMPLE 4

A batch of tablets were prepared by direct compression. Each tablet had the following composition:-

| | |
|---|---|
| 5 mg | selegiline hydrochloride |
| 49.45 mg | Direct Compression Lactose |
| 24 mg | Microcrystalline Cellulose |
| 11 mg | Maize Starch |
| 0.55 mg | Zinc Stearate |

### The tablets gave an assay of 100.7%

From extensive research and development in producing tablets of selegiline by direct compression techniques, we have noticed a very significant interaction between selegiline hydrochloride and conventional bulking agents such as DC Lactose and microcrystalline cellulose. It has not been possible using conventional formulations to produce a tablet of selegiline by direct compression without a very significant adverse affect on the assay results for the tablets produced. In all cases, the assay was found to be less than 95%, in some cases, as low as 70%. It is not fully understood why such poor assay results were obtained. It is, however, speculated that there may be an interaction between the selegiline and the DC Lactose and microcrystalline cellulose, resulting possibly in the formation of a complex which adversely affects the amount of selegiline detectable or available on assay. We have surprisingly found that the interaction is avoided and good assay results are obtained if an inhibitor, particularly maize starch, is incorporated especially in the amounts mentioned above.

It will be appreciated that it may be possible to use direct compression excipients either in addition to or as an alternative either to direct compression lactose or microcrystalline cellulose. Such direct compression excipients include calcium hydrogen phosphate, compressible sugar, dextrates, and pregelatinized starch.

It will be appreciated that the formulation may include any suitable lubricant such as magnesium stearate or zinc stearate as described above or other stearic acid salts such as calcium stearate. Other suitable lubricants may also be incorporated, including glyceryl monostearate, glyceryl palmitostearate, hydrogenated castor oil, hydrogenated vegetable oil type 1, light mineral oil, mineral oil, polyethylene glycol, sodium benzoate, sodium lauryl sulphate, sodium stearyl fumarate, stearic acid and talc.

It will also be appreciated that while the presence of an antioxidant may not be essential, in cases where such an antioxidant is used it may be selected from one or more of the following: alpha tocopherol, ascorbic acid, ascorbyl palmitate, butylated hydroxyanisole, butylated hydroxytoluene, citric acid, fumaric acid, malic acid, propylgallate, sodium ascorbate, sodium metabisulphite.

We have also found that the interactions described above also apply to other active pharmaceutical ingredients. The following examples illustrate how similar techniques may also be applied to direct compression pharmaceutical tablet formulations of active ingredients other than selegiline.

### EXAMPLE B - COMPARISON

A batch of tablets were prepared by direct compression. Each tablet had the following composition:-

| | |
|---|---|
| 5 mg | oxybutynin chloride |
| 174 mg | DC Lactose |
| 1 mg | Magnesium Stearate |

The tablets gave an assay of 88.3%.

### EXAMPLE 5

A batch of tablets were prepared by direct compression. Each tablet had the following composition:-

| | |
|---|---|
| 5 mg | oxybutynin chloride |
| 119 mg | DC Lactose |
| 43 mg | microcrystalline cellulose |
| 12 mg | crospovidone |
| 1 mg | magnesium stearate |

The tablets gave an assay of 97.9%.

### EXAMPLE C - COMPARISON

A batch of tablets were prepared by direct compression. Each tablet had the following composition.

| | |
|---|---|
| 5 mg | bumetanide |
| 343 mg | DC Lactose |
| 2 mg | magnesium Stearate |

The tablets gave an assay of 91%.

### EXAMPLE D - COMPARISON

A batch of tablets were prepared by direct compression. Each tablet had the following composition.

| | |
|---|---|
| 5 mg | bumetanide |
| 343 mg | microcrystaline cellulose |
| 2 mg | magnesium stearate |

The tablets gave a assay of 93.7%.

### EXAMPLE 6

A batch of tablets were prepared by direct compression. Each tablet had the following composition.

| | |
|---|---|
| 5 mg | bumetanide |
| 237.5 mg | DC Lactose |
| 80 mg | microcrystalline cellulose |
| 25 mg | crospovidone |
| 2.5 mg | magnesium stearate |

The tablets gave an assay of 97.4%.

### EXAMPLE 7

A batch of tablets were prepared by direct compression. Each tablet had the following composition.

| | |
|---|---|
| 5 mg | bumetanide |
| 237.5 mg | DC Lactose |
| 80 mg | microcrystalline cellulose |
| 25 mg | maize starch |
| 2.5 mg | magnesium stearate |

The tablets gave an assay of 98.2%.

### EXAMPLE 8

A batch of tablets were prepared by direct compression. Each tablet had the following composition:-

| | |
|---|---|
| 2.5 mg | indapamide hemihydrate |
| 82.75 mg | DC Lactose |
| 4 mg | carboxymethyl starch |
| 0.75 mg | magnesium stearate. |

The tablets gave an assay of 100.4%.

It will be appreciated that similar techniques may be applied to produce direct compression tablet formulations of other active pharmaceutical ingredients which interact with direct compression excipients.

The invention is not limited to the embodiments hereinbefore described which may be varied in detail.

## Claims

1. A method of preparing a pharmaceutical tablet comprising:
preparing a formulation by dry blending an active pharmaceutical ingredient selected from selegiline, oxybutynin, bumetanide, indapamide and pharmaceutically acceptable salts thereof, a direct compression excipient capable of interacting with the active pharmaceutical ingredient and reducing the amount thereof detectable in the pharmaceutical tablet, and an amount of an inhibitor selected from maize starch, a crospovidone based product and carboxymethyl starch, sufficient to reduce said interaction;
and forming said tablet from said formulation by direct compression.

2. A method of preparing a pharmaceutical tablet comprising:
preparing a formulation by dry blending a direct compression excipient comprising direct compression lactose or microcrystalline cellulose, an active pharmaceutical ingredient capable of interacting with direct compression lactose or microcrystalline cellulose such that the amount of said active pharmaceutical ingredient detectable in the pharmaceutical tablet is reduced, and an amount of an inhibitor selected from maize starch, a crospovidone based product, and carboxymethyl starch sufficient to reduce said interaction;
and forming said tablet from said formulation by direct compression.

3. A method as claimed in claim 1, wherein the excipient comprises direct compression lactose or microcrystalline cellulose.

4. A method as claimed in any of claims 1-3 wherein the formulation contains an amount of the inhibitor to substantially prevent the interaction between the direct compression excipient and the active pharmaceutical ingredient.

5. A method as claimed in any of claims 1-4 wherein the weight ratio of inhibitor to the active pharmaceutical ingredient in the formulation is from 1:50 to 4:1.

6. A method as claimed in any of claims 1-5 wherein the weight ratio of inhibitor to the active pharmaceutical ingredient in the formulation is from 8:5 to 12:5.

7. A method as claimed in any preceding claim wherein the inhibitor includes a maize starch.

8. A method as claimed in any proceeding claim in which the inhibitor includes a crospovidone based product.

9. A method as claimed in any of claims 1-8 wherein the formulation comprises direct compression lactose and the weight ratio of direct compression lactose to the active pharmaceutical ingredient in the formulation is from 2:1 to 100:1.

10. A method as claimed in claim 9 wherein the weight ratio of direct compression lactose to the active pharmaceutical ingredient in the formulation is from 7:1 to 50:1.

11. A method as claimed in claim 9 or claim 10 wherein the weight ratio of direct compression lactose to the active pharmaceutical ingredient in the formulation is from 8:11 to 11:1.

12. A method as claimed in any of claims 1 to 8, wherein the formulation comprises microcrystalline cellulose and the weight ratio of microcrystalline cellulose to active pharmaceutical ingredient in the formulation is from 2:1 to 100:1.

13. A method as claimed in claim 12 wherein the weight ratio of microcrystalline cellulose to active pharmaceutical ingredient in the formulation is from 3:1 to 17:1.

14. A method as claimed in claim 12 or 13 wherein the weight ratio of microcrystalline cellulose to active pharmaceutical ingredient in the formulation is from 4:1 to 16:1.

15. A method as claimed in any preceding claim wherein the formulation includes a lubricant.

16. A method as claimed in claim 15 wherein the lubricant is present in an amount from 0.1% to 5% by weight of the formulation.

17. A method as claimed in claim 16 wherein the lubricant is present in an amount of approximately 0.25% by weight of the formulation.

18. A method as claimed in any preceding claim wherein the formulation includes an antioxidant or other suitable stabiliser to enhance the stability of the tablet.

19. A method as claimed in claim 18 wherein the antioxidant is present in an amount from 0.5% to 2% by weight of the formulation.

20. A method as claimed in any proceeding claim wherein the formulation includes either a glidant, a disintegrant, a filler, a wetting agent, a stabiliser, a binder or any combination of these materials.

21. A method as claimed in any preceding claim wherein the active pharmaceutical ingredient is selegiline or a pharmaceutically acceptable salt thereof.

22. A method as claimed in claim 21, wherein the active pharmaceutical ingredient is selegiline hydrochloride.

23. A method as claimed in any of claims 1-20 wherein the active pharmaceutical ingredient is oxybutynin or a pharmaceutically acceptable salt thereof.

24. A method as claimed in claim 23, wherein the active pharmaceutical ingredient is oxybutynin chloride.

25. A method as claimed in any of claims 1-20 wherein the active pharmaceutical ingredient is bumetanide or a pharmaceutically acceptable salt thereof.

26. A method as claimed in any of claims 1-20 wherein the active pharmaceutical ingredient is indapamide or a pharmaceutically acceptable salt thereof.

27. A method as claimed in claim 26, wherein the active pharmaceutical ingredient is indapamide hemihydrate.

28. A method as claimed in claim 1 or claim 2, wherein the formulation includes a lubricant and the method includes the step of mixing the ingredients except the lubricant; subsequently adding the lubricant; and further mixing the ingredients.

29. A tablet of an active pharmaceutical ingredient prepared by a method as claimed in any of claims 1-28.

30. A tablet of selegiline or a pharmaceutically acceptable salt thereof, prepared by a method as claimed in any of claims 1-22 and 28.

31. A table of oxybutynin or a pharmaceutically acceptable salt thereof, prepared by a method as claimed in any of claims 1-20, 23, 24 and 28.

32. A tablet of bumetanide or a pharmaceutically acceptable salt thereof prepared by a method as claimed in any of claims 1-20, 25 and 28.

33. A tablet of indapamide or a pharmaceutically acceptable salt thereof, whenever produced by a method as claimed in any of claims 1-20 and 26-28.

## Patentansprüche

1. Verfahren zur Herstellung einer pharmazeutischen Tablette, umfassend:
Zubereiten einer Formulierung durch trockenes Mischen einer aktiven pharmazeutischen Zutat ausgewählt aus Selegilin, Oxybutynin, Bumetanid, Indapamid und pharmazeutisch akzeptablen Salzen davon, einer Trägersubstanz zur Direktverpressung, die in der Lage ist, mit der aktiven pharmazeutischen Zutat zu wechselwirken und die davon in der pharmazeutischen Tablette detektierbare Menge zu reduzieren, und einer Menge eines Hemmstoffes, ausgewählt aus Maisstärke, einem auf Crospovidon basierenden Produkt und Carboxymethylstärke, die ausreicht, um die genannte Wechselwirkung zu reduzieren;
und Herstellen der Tablette aus der Formulierung durch Direktverpressen.

2. Verfahren zur Herstellung einer pharmazeutischen Tablette, umfassend:
Zubereiten einer Formulierung durch trockenes Mischen einer Trägersubstanz zur Direktverpressung umfassend Laktose zur Direktverpressung oder mikrokristalline Zellulose, einer aktiven pharmazeutischen Zutat, die in der Lage ist, mit Laktose zur Direktverpressung oder mikrokristalliner Zellulose zu wechselwirken, so daß die in der pharmazeutischen Tablette detektierbare Menge der genannten aktiven pharmazeutischen Zutat reduziert ist, und einer Menge eines Hemmstoffes, ausgewählt aus Maisstärke, einem auf Crospovidon basierenden Produkt und Carboxymethylstärke, die ausreicht, um die genannte Wechselwirkung zu reduzieren;
und Herstellen der Tablette aus der Formulierung durch Direktverpressen.

3. Verfahren nach Anspruch 1, wobei die Trägersubstanz Laktose zur Direktverpressung oder mikrokristalline Zellulose umfaßt.

4. Verfahren nach einem der Ansprüche 1-3, wobei die Formulierung eine Menge des Hemmstoffes enthält, die die Wechselwirkung zwischen der Trägersubstanz zur Direktverpressung und der aktiven pharmazeutischen Zutat im wesentlichen verhindert.

5. Verfahren nach einem der Ansprüche 1-4, wobei das Gewichtsverhältnis des Hemmstoffes zu der aktiven pharmazeutischen Zutat in der Formulierung 1:50 bis 4:1 beträgt.

6. Verfahren nach einem der Ansprüche 1-5, wobei das Gewichtsverhältnis des Hemmstoffes zu der aktiven pharmazeutischen Zutat in der Formulierung 8:5 bis 12:5 beträgt.

7. Verfahren nach einem der vorhergehenden Ansprüche, wobei der Hemmstoff eine Maisstärke enthält.

8. Verfahren nach einem der vorhergehenden Ansprüche, wobei der Hemmstoff ein auf Crospovidon basierendes Produkt enthält.

9. Verfahren nach einem der Ansprüche 1-8, wobei die Formulierung Laktose zur Direktverpressung umfaßt und das Gewichtsverhältnis der Laktose zur Direktverpressung zu der aktiven pharmazeutischen Zutat in der Formulierung 2:1 bis 100:1 beträgt.

10. Verfahren nach Anspruch 9, wobei das Gewichtsverhältnis der Laktose zur Direktverpressung zu der aktiven pharmazeutischen Zutat in der Formulierung 7:1 bis 50:1 beträgt.

11. Verfahren nach Anspruch 9 oder 10, wobei das Gewichtsverhältnis der Laktose zur Direktverpressung zu der aktiven pharmazeutischen Zutat in der Formulierung 8:11 bis 11:1 beträgt.

12. Verfahren nach einem der Ansprüche 1 bis 8, wobei die Formulierung mikrokristalline Zellulose umfaßt und das Gewichtsverhältnis der mikrokristallinen Zellulose zu der aktiven pharmazeutischen Zutat in der Formulierung 2:1 bis 100:1 beträgt.

13. Verfahren nach Anspruch 12, wobei das Gewichtsverhältnis der mikrokristallinen Zellulose zu der aktiven pharmazeutischen Zutat in der Formulierung 3:1 bis 17:1 beträgt.

14. Verfahren nach Anspruch 12 oder 13, wobei das Gewichtsverhältnis der mikrokristallinen Zellulose zu der aktiven pharmazeutischen Zutat in der Formulierung 4:1 bis 16:1 beträgt.

15. Verfahren nach einem der vorhergehenden Ansprüche, wobei die Formulierung ein Schmiermittel enthält.

16. Verfahren nach Anspruch 15, wobei das Schmiermittel in einer Menge von 0,1% bis 5% vom Gewicht der Formulierung vorhanden ist.

17. Verfahren nach Anspruch 16, wobei das Schmiermittel in einer Menge von etwa 0,25% vom Gewicht der Formulierung vorhanden ist.

18. Verfahren nach einem der vorhergehenden Ansprüche, wobei die Formulierung ein Antioxidans oder andere geeignete Stabilisatoren zur Verbesserung der Stabilität der Tablette enthält.

19. Verfahren nach Anspruch 18, wobei das Antioxidans in einer Menge von 0,5% bis 2% vom Gewicht der Formulierung vorhanden ist.

20. Verfahren nach einem der vorhergehenden Ansprüche, wobei die Formulierung entweder ein Gleitmittel, ein Sprengmittel, ein Füllmittel, ein Benetzungsmittel, einen Stabilisator, ein Bindemittel oder jede Kombination dieser Materialien enthält.

21. Verfahren nach einem der vorhergehenden Ansprüche, wobei die aktive pharmazeutische Zutat Selegilin oder ein pharmazeutisch akzeptables Salz davon ist.

22. Verfahren nach Anspruch 21, wobei die aktive pharmazeutische Zutat Selegilinhydrochlorid ist.

23. Verfahren nach einem der Ansprüche 1-20, wobei die aktive pharmazeutische Zutat Oxybutynin oder ein pharmazeutisch akzeptables Salz davon ist.

24. Verfahren nach Anspruch 23, wobei die aktive pharmazeutische Zutat Oxybutyninchlorid ist.

25. Verfahren nach einem der Ansprüche 1-20, wobei die aktive pharmazeutische Zutat Bumetanid oder ein pharmazeutisch akzeptables Salz davon ist.

26. Verfahren nach einem der Ansprüche 1-20, wobei die aktive pharmazeutische Zutat Indapamid oder ein pharmazeutisch akzeptables Salz davon ist.

27. Verfahren nach Anspruch 26, wobei die aktive pharmazeutische Zutat Indapamid-Hemihydrat ist.

28. Verfahren nach Anspruch 1 oder 2, wobei die Formulierung ein Schmiermittel enthält und das Verfahren den Schritt des Mischens der Inhaltsstoffe außer dem Schmiermittel, der anschließenden Zugabe des Schmiermittels und des weiteren Mischens der Inhaltsstoffe beinhaltet.

29. Tablette mit einer aktiven pharmazeutischen Zutat, hergestellt nach einem der Verfahren gemäß einem der Ansprüche 1-28.

30. Tablette mit Selegilin oder einem pharmazeutisch akzeptablen Salz davon, hergestellt nach einem der Verfahren gemäß einem der Ansprüche 1-22 und 28.

31. Tablette mit Oxybutynin oder einem pharmazeutisch akzeptablen Salz davon, hergestellt nach einem der Verfahren gemäß einem der Ansprüche 1-20, 23, 24 und 28.

32. Tablette mit Bumetanid oder einem pharmazeutisch akzeptablen Salz davon, hergestellt nach einem der Verfahren gemäß einem der Ansprüche 1-20, 25 und 28.

33. Tablette mit Indapamid oder einem pharmazeutisch akzeptablen Salz davon, hergestellt nach einem der Verfahren gemäß einem der Ansprüche 1-20 und 26-28.

## Revendications

1. Une méthode pour la préparation d'un comprimé pharmaceutique, méthode consistant à :
- préparer une formulation par mélangeage à sec d'un ingrédient pharmaceutiquement actif choisi entre la sélégiline, l'oxybutynine, la bumétanide, l'indapamide et leurs sels pharmaceutiquement acceptables, un excipient pour compression directe susceptible d'interagir avec l'ingrédient pharmaceutiquement actif et d'en réduire la quantité détectable dans le comprimé pharmaceutique et une quantité d'un inhibiteur choisi entre l'amidon de maïs, un produit à base de crospovidone et un amidon carboxyméthylé, suffisants pour réduire ladite interaction ;
- et former ledit comprimé à partir de ladite formulation par une technique de compression directe.

2. Une méthode pour la préparation d'un comprimé pharmaceutique, méthode consistant à :
- préparer une formulation par mélangeage à sec d'un excipient pour compression directe consistant en lactose pour compression directe ou en cellulose microcristalline, un ingrédient pharmaceutiquement actif susceptible d'interagir avec ledit lactose pour compression directe ou ladite cellulose microcristalline et tel que la quantité dudit ingrédient pharmaceutiquement actif détectable dans le comprimé pharmaceutique soit réduite, et une quantité d'un inhibiteur choisi entre l'amidon de maïs, un produit à base de crospovidone et un amidon carboxyméthylé, suffisantes pour réduire ladite interaction ;
- et former ledit comprimé à partir de ladite formulation par une technique de compression directe.

3. Une méthode selon la revendication 1, dans laquelle l'excipient consiste en lactose pour compression directe ou en cellulose microcristalline.

4. Une méthode selon l'une quelconque des revendications 1-3, dans laquelle la formulation contient une quantité de l'inhibiteur empêchant pratiquement l'interaction entre l'excipient pour compression directe et l'ingrédient pharmaceutiquement actif.

5. Une méthode selon l'une quelconque des revendications 1-4, dans laquelle la proportion en poids de l'inhibiteur par rapport à l'ingrédient pharmaceutiquement actif dans la formulation est de 1:50 à 4:1.

6. Une méthode selon l'une quelconque des revendications 1-5, dans laquelle la proportion en poids de l'inhibiteur par rapport à l'ingrédient pharmaceutiquement actif dans la formulation est de 8:5 à 12:5.

7. Une méthode selon l'une quelconque des revendications précédentes, dans laquelle l'inhibiteur contient de l'amidon de maïs.

8. Une méthode selon l'une quelconque des revendications précédentes, dans laquelle l'inhibiteur contient un produit à base de crospovidone.

9. Une méthode selon l'une quelconque des revendications 1-8, dans laquelle la formulation comprend du lactose pour compression directe et le rapport en poids du lactose pour compression directe par rapport à l'ingrédient pharmaceutiquement actif de la formulation est de 2:1 à 100:1.

10. Une méthode selon la revendication 9, dans laquelle la proportion en poids du lactose pour compression directe par rapport à l'ingrédient pharmaceutiquement actif dans la formulation est de 7:1 à 50:1.

11. Une méthode selon la revendication 9 ou la revendication 10, dans laquelle la proportion en poids du lactose pour compression directe par rapport à l'ingrédient pharmaceutiquement actif dans la formulation est de 8:11 à 11:1.

12. Une méthode selon l'une quelconque des revendications 1-8, dans laquelle la formulation contient de la cellulose microcristalline et la proportion en poids de cellulose microcristalline par rapport à l'ingrédient pharmaceutiquement actif de la formulation est de 2:1 à 100:1.

13. Une méthode selon la revendication 12, dans laquelle la proportion en poids de cellulose microcristalline par rapport à l'ingrédient pharmaceutiquement actif dans la formulation est de 3:1 à 17:1.

14. Une méthode selon la revendication 12 ou 13, dans laquelle la proportion en poids de cellulose microcristalline par rapport à l'ingrédient pharmaceutiquement actif dans la formulation est de 4:1 à 16:1.

15. Une méthode selon l'une quelconque des revendications précédentes, dans laquelle la formulation contient un lubrifiant.

16. Une méthode selon la revendication 15, dans laquelle le lubrifiant est présent à raison d'une quantité de 0,1% à 5% en poids de la formulation.

17. Une méthode selon la revendication 16, dans laquelle le lubrifiant est présent à raison d'une quantité d'approximativement 0,25% en poids de la formulation.

18. Une méthode selon l'une quelconque des revendications précédentes, dans laquelle la formulation contient un antioxydant ou autre stabilisant approprié pour accroître la stabilité du comprimé.

19. Une méthode selon la revendication 18, dans laquelle l'antioxydant est présent à raison de 0,5% à 2% en poids de la formulation.

20. Une méthode selon l'une quelconque des revendications précédentes, dans laquelle la formulation contient soit un agent de glissement, un désintégrant, une charge, un agent mouillant, un stabilisant, un liant ou toute combinaison de ces matières.

21. Une méthode selon l'une quelconque des revendications précédentes, dans laquelle l'ingrédient pharmaceutiquement actif est la sélégiline ou un sel pharmaceutiquement acceptable de celle-ci.

22. Une méthode selon la revendication 21, dans laquelle l'ingrédient pharmaceutiquement actif est le chlorhydrate de sélégiline.

23. Une méthode selon l'une quelconque des revendications 1-20, dans laquelle l'ingrédient pharmaceutiquement actif est l'oxybutynine ou un sel pharmaceutiquement acceptable de celle-ci.

24. Une méthode selon la revendication 23, dans laquelle l'ingrédient pharmaceutiquement actif est le chlorure d'oxybutynine.

25. Une méthode selon l'une quelconque des revendications 1-20, dans laquelle l'ingrédient pharmaceutiquement actif est la bumétanide ou un sel pharmaceutiquement acceptable de celle-ci.

26. Une méthode selon l'une quelconque des revendications 1-20, dans laquelle l'ingrédient pharmaceutiquement actif est l'indapamide ou un sel pharmaceutiquement acceptable de celle-ci.

27. Une méthode selon la revendication 26, dans laquelle l'ingrédient pharmaceutiquement actif est l'hémihydrate d'indapamide.

28. Une méthode selon la revendication 1 ou la revendication 2, dans laquelle la formulation contient un lubrifiant et la méthode comprend l'étape de mélangeage des ingrédients à l'exception du lubrifiant puis ultérieurement l'addition du lubrifiant ; et un nouveau mélangeage des ingrédients.

29. Un comprimé d'un ingrédient pharmaceutiquement actif préparé par une méthode selon l'une quelconque des revendications 1-28.

30. Un comprimé de sélégiline ou d'un sel pharmaceutiquement acceptable de celle-ci, préparé par une méthode selon l'une quelconque des revendications 1-22 et 28.

31. Un comprimé d'oxybutynine ou d'un sel pharmaceutiquement acceptable de celle-ci, préparé par une méthode selon l'une quelconque des revendications 1-20, 23, 24 et 28.

32. Un comprimé de bumétanide ou d'un sel pharmaceutiquement acceptable de celle-ci, préparé par une méthode selon l'une quelconque des revendications 1-20, 25 et 28.

33. Un comprimé d'indapamide ou d'un sel pharmaceutiquement acceptable de celle-ci, préparé par une méthode selon l'une quelconque des revendications 1-20 et 26-28.
